(19) Europäisches Patentamt
European Patent Office
Office européen des brevets

(11) Veröffentlichungsnummer: **0 120 257**
**B1**

# (12) EUROPÄISCHE PATENTSCHRIFT

(45) Veröffentlichungstag der Patentschrift: **17.12.86**

(51) Int. Cl.⁴: **C 07 D 311/76,** C 07 D 311/92, C 07 D 311/94

(21) Anmeldenummer: **84101471.5**

(22) Anmeldetag: **13.02.84**

## (54) Verfahren zur Herstellung von Isochroman-Derivaten.

(30) Priorität: **23.02.83 DE 3306200**

(43) Veröffentlichungstag der Anmeldung: **03.10.84 Patentblatt 84/40**

(45) Bekanntmachung des Hinweises auf die Patenterteilung: **17.12.86 Patentblatt 86/51**

(84) Benannte Vertragsstaaten: **CH DE FR GB IT LI NL**

(56) Entgegenhaltungen:
**DE-C-915 811**
**US-A-3 978 090**

**Die Akte enthält technische Angaben, die nach dem Eingang der Anmeldung eingereicht wurden und die nicht in dieser Patentschrift enthalten sind.**

(73) Patentinhaber: **Haarmann & Reimer GmbH, Postfach 1253, D-3450 Holzminden (DE)**

(72) Erfinder: **Finkelmeier, Horst, Dr., Dr. Jasper-Strasse 26, D-3450 Holzminden (DE)**
Erfinder: **Hopp, Rudolf, Dr., Auf dem Gehrenkamp 28, D-3450 Holzminden (DE)**

(74) Vertreter: **Mann, Volker, Dr., c/o Bayer AG Konzernverwaltung RP Patentabteilung, D-5090 Leverkusen 1, Bayerwerk (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 120 257 B1

LIBER, STOCKHOLM 1986

**Beschreibung**

Die Erfindung betrifft ein Verfahren zur Herstellung von Isochroman-Derivaten.

Aus Chem. Ber. 89, 1254 (1956) ist bekannt, Isochroman durch Umsetzung von ß-Phenyl-ethylalkohol mit Formaldehyd in Gegenwart von Chlorwasserstoff mit einer Ausbeute von 50 % herzustellen.

In der DE-OS 19 51 001 wird beschrieben, Isochromane herzustellen, indem man einen aromatischen Kohlenwasserstoff mit einem niedrigen Alkylenoxid in Gegenwart von Aluminiumchlorid umsetzt, um einen Aralkanol/Aluminiumchlorid-Komplex herzustellen, man dann das Aluminiumchlorid mit einem freie Hydroxylgruppen enthaltenden Stoff teilweise deaktiviert, und man schließlich Formaldehyd zusetzt, um das Aralkanol zu einem Isochroman zu cyclisieren.

Nach der DE-OS 25 16 809 können Isochromane durch Umsetzung von ß-Phenyl-ethylalkohol mit einem Acetal in Gegenwart von Protonensäuren hergestellt werden.

Das Verfahren liefert Isochromane in unbefriedigenden Ausbeuten. Bei dem Verfahren nach der DE-OS 25 39 773, bei dem zusätzlich ein Azeotropiermittel, wie n-Hexan, Cyclohexan, Methylcyclohexan, Benzol oder Toluol, zugesetzt wird, ist die Aufarbeitung durch das Azeotropiermittel sehr aufwendig.

Es wurde ein Verfahren zur Herstellung von Isochroman-Derivaten gefunden, daß dadurch gekennzeichnet ist, daß man Alkohole der Formel (I)

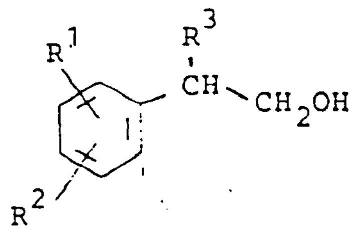

(I)

in der

$R^1$ und $R^2$ gleich oder verschieden sind und Wasserstoff, Niederalkyl oder Niederalkoxy bedeuten oder gemeinsam einen gegebenenfalls durch Niederalkyl substituierten Cyclopentan- oder Cyclohexanring bilden und

$R^3$ Wasserstoff oder Niederalkyl bedeutet,

mit Formaldehyd und einem Carbonsäureanhydrid der Formel (II)

$$R^4-\overset{O}{\overset{\|}{C}}-O-\overset{O}{\overset{\|}{C}}-R^4 \qquad (II)$$

in der

$R^4$ für einen Niederalkylrest steht,

oder mit einem Methylen-diester der Formel (III)

$$R^4-\overset{O}{\overset{\|}{C}}-O-CH_2-O-\overset{O}{\overset{\|}{C}}-R^4 \qquad (III)$$

in der

$R^4$ für einen Niederalkylrest steht,

in Gegenwart eines sauren Katalysators bei erhöhter Temperatur umsetzt.

Nach dem erfindungsgemäßen Verfahren lassen sich Isochromane mit hohen Ausbeuten und in einfacher Weise herstellen.

Das erfindungsgemäße Verfahren kann anhand der folgenden Reaktionsgleichung erläutert werden:

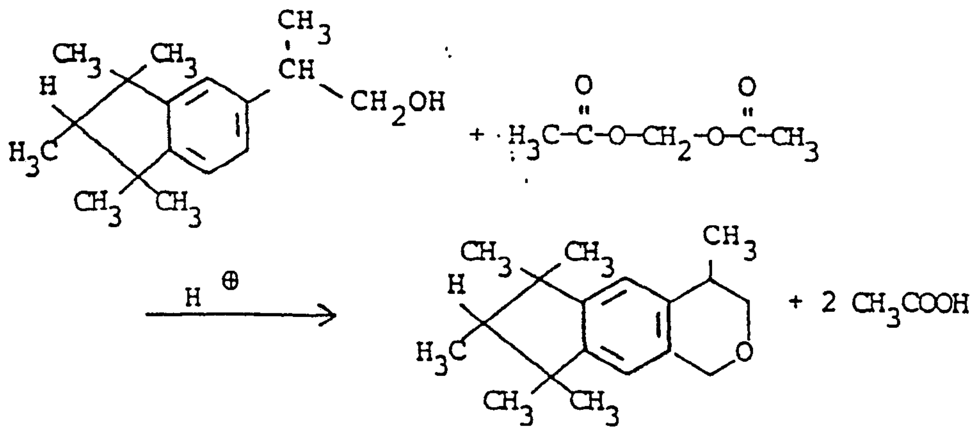


Niederalkyl bedeutet erfindungsgemäß einen geradkettigen oder verzweigten Kohlenwasserstoffrest mit 1 bis etwa 6 Kohlenstoffatomen. Beispielsweise seien die folgenden Niederalkylreste genannt: Methyl, Ethyl, Propyl, Isopropyl, Butyl, Isobutyl, Pentyl, Isopentyl, Hexyl und Isohexyl. Bevorzugte Niederalkylreste sind der Methyl-, Ethyl- und Isopropylrest.

Niederalkoxy enthält erfindungsgemäß im Alkylteil einen geradkettigen oder verzweigten Kohlenwasserstoffrest mit 1 bis etwa 6 Kohlenstoffatomen. Beispielsweise seien die folgenden Niederalkoxyreste genannt: Methoxy, Ethoxy, Propoxy, Isopropoxy, Butoxy, Isobutoxy, Pentoxy, Isopentoxy, Hexoxy und Isohexoxy. Bevorzugte Niederalkoxyreste sind der Methoxy-, der Ethoxy- und der Isopropoxyrest.

Die Reste $R^1$ und $R^2$ können zu einem Cyclopentan- oder zu einem Cyclohexanrest verbunden sein. Der Cyclopentanbzw. der Cyclohexanrest kann sowohl in 2,3- als auch in 3,4-Stellung an den Phenylrest annelliert sein. Der Cyclopentan- bzw. der Cyclohexanrest kann erfindungsgemäß bevorzugt durch Niederalkylgruppen, bevorzugt bis zu 6, insbesondere bevorzugt 3 bis 6, substituiert sein.

Bevorzugte Alkohole für das erfindungsgemäße Verfahren sind Verbindungen der Formel (IV)

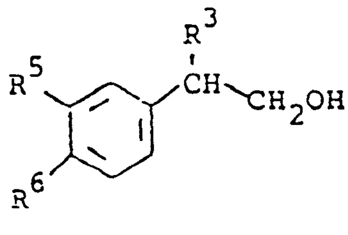
(IV)

in der

$R^5$ und $R^6$ jeweils Wasserstoff bedeuten oder gemeinsam einen durch Niederalkyl substituierten Cyclopentan- oder Cyclohexanring bilden und

$R^3$ die obengenannte Bedeutung hat.

Alkohole für das erfindungsgemäße Verfahren sind an sich bekannt (DE-AS 12 87 242, DE-AS 22 19 953, JP 53 031 635).

Sie können beispielsweise durch Umsetzung von Aromaten mit einem niederen Alkylenoxid ($C_1$ bis etwa $C_6$) in Gegenwart von Aluminiumchlorid hergestellt werden.

Formaldehyd für das erfindungsgemäße Verfahren ist im wesentlichen Paraformaldehyd.

Carbonsäureanhydride für das erfindungsgemäße Verfahren sind beispielsweise Essigsäureanhydrid, Propionsäureanhydrid, n-Buttersäureanhydrid, Isobuttersäureanhydrid, n-Valeriansäureanhydrid, Isovaleriansäureanhydrid und n-Capronsäureanhydrid. Bevorzugtes Anhydrid ist das Essigsäureanhydrid.

Methylendiester für das erfindungsgemäße Verfahren können durch Umsetzung von Paraformaldehyd mit einem Carbonsäureanhydrid hergestellt werden (Liebigs Ann. Chem. 402, 127 (1913)).

Die Herstellung kann beispielsweise durchgeführt werden, indem man die Reaktionspartner in Gegenwart katalytischer Mengen einer Säure erhitzt.

Als Methylendiester seien beispielsweise genannt: Methylen-diacetat, Methylen-dipropionat, Methylen-dinbutyrat, Methylen-di-isobutyrat, Methylen-dicapronat.

Bevorzugter Methylendiester ist das Methylen-diacetat.

Als saure Katalysatoren eignen sich beispielsweise saure Tone, wie z.B. sauer dotierte Montmorillonite, p-Toluolsulfonsäure oder ortho-Phosphorsäure. Für das erfindungsgemäße Verfahren werden Phosphorsäure oder saure Tone als saurer Katalysator bevorzugt.

Das erfindungsgemäße Verfahren kann im Temperaturbereich von Raumtemperatur bis 200° C durchgeführt werden. Bevorzugt wird eine Temperatur von 70 bis 180° C, insbesondere bevorzugt von 100 bis 150° C.

Das erfindungsgemäße Verfahren kann sowohl bei Normaldruck als auch bei Überdruck durchgeführt werden. Beispielsweise sei ein Druckbereich von Normaldruck bis 20 bar genannt. Für das erfindungsgemäße Verfahren wird ein Druck von 1,2 bis 10 bar bevorzugt.

Das erfindungsgemäße Verfahren kann beispielsweise wie folgt durchgeführt werden: Die Reaktanden werden vorzugsweise in einem Autoklaven vorgelegt und bei der Reaktionstemperatur gerührt. Dann destilliert man die bei der Reaktion entstandene Carbonsäure ab. Das Reaktionsprodukt wird in einem organischen Lösungsmittel, wie Toluol, aufgenommen und mit einer alkalischen wäßrigen Lösung, wie Natronlauge oder Sodalösung, neutralisiert oder gewaschen. Das Reaktionsprodukt wird im Vakuum destilliert. Im Falle der sauren Tone wird der Katalysator abfiltriert und das Filtrat direkt destilliert.

Nach dem erfindungsgemäßen Verfahren können Isochroman-Derivate der Formel (V)

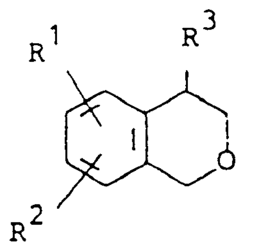

(V)

in der

$R^1$ bis $R^3$ die obengenannte Bedeutung haben,

hergestellt werden.

Beispielsweise seien die folgenden Isochromane genannt:

6-Oxa-1,1,2,3,3,8-hexamethyl-2,3,5,6,7,8-hexahydro-1H-benz[f]inden (Formel VI); 6-Oxa-1,1,3,8-tetramethyl-3-ethyl-2,3,5,6,7,8-hexahydro-1H-benz[f]inden (Formel VII); 6-Oxa-1,3,3,8-tetramethyl-1-ethyl-2,3,5,6,7,8-hexahydro-1H-benz[f]inden (Formel VIII); 6-Oxa-1,1,2,3,3-penta-methyl-2,3,5,6,7,8-hexahydro-1H-benz[f]inden (Formel IX); 2-Oxa-4,5,5,8,8-pentamethyl-1,2,3,4,5,6,7,8-octahydroan-thracen (Formel X); 2-Oxa-5,5,8,8-tetramethyl-1,2,3,4,5,6,7,8-octahydroanthracen (Formel XI); 6-Oxa-1,1,2,8-tetra-methyl-3-isopropyl-2,3,5,6,7,8-hexahydro-1H-benz[f]inden (Formel XII); 6-Oxa-2,3,3,8-tetramethyl-1-isopropyl-2,3,5,6,7,8-hexahydro-1H-benz[f]inden (Formel XIII) 6-Oxa-1,1,2-trimethyl-3-isopropyl-2,3,5,6,7,8-hexahydro-1H-benz[f]inden (Formel XIV) und 6-Oxa-2,3,3-trimethyl-1-isopropyl-2,3,5,6,7,8-hexahydro-1H-benz[f]inden (Formel XV).

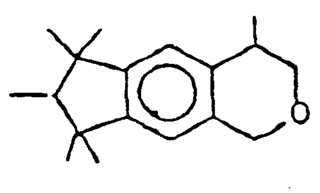

(VI)

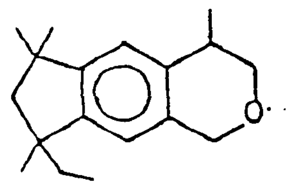

(VII)

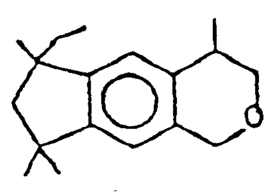

(VIII)

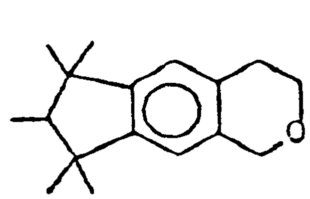

(IX)

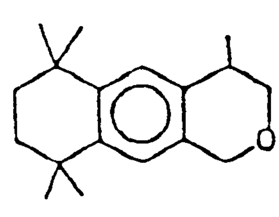

(X)

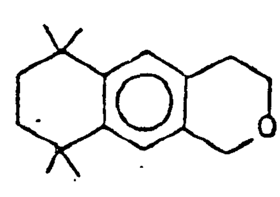

(XI)

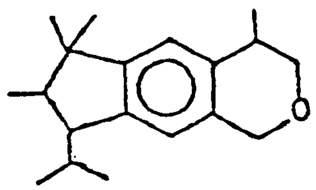

(XII)

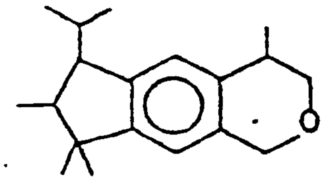

(XIII)

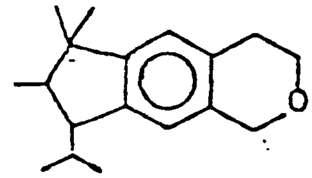

(XIV)

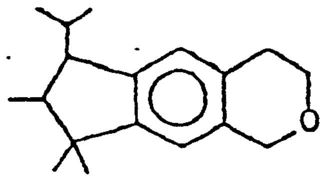

(XV)

Die erfindungsgemäßen Isochromane mit annellierten Polyalkylcyclopentan- und Polyalkylcyclohexanringen sind hochwertige Moschus-Riechstoffe.

Für den Fall, daß nach dem erfindungsgemäßen Verfahren Isochromane ohne annellierten Cyclopentan- bzw. Cyclohexanring hergestellt wurden, ist es möglich, diesen noch in einem zweiten Reaktionsschritt einzuführen und so auch die Moschus-Riechstoffe zu erhalten. So kann man Isochromane, bei denen zwei benachbare Kohlenstoffatome im Phenylring unsubstituiert sind, mit geeigneten Dihalogeniden, Diolen oder Dienen umsetzen und so den Cyclopentan- bzw. Cyclohexanring annellieren (DE-AS 12 87 242, Spalte 4, Zeilen 12-48).

**Beispiel 1**

6-Oxa-1,1,2,3,3,8-hexamethyl-2,3,5,6,7,8-hexahydro-1H-benz[f]inden

Man gibt 94 g Essigsäureanhydrid, 50 g 85 %ige Phosphorsäure, 27,6 g Paraformaldehyd und 200 g 2-(1',1',2',3',3'-Pentamethyl-5'-indanyl)-1-propanol nacheinander in einen Autoklaven und erwärmt auf 135°C. Man rührt 1 h bei dieser Temperatur und destilliert danach die entstandene Essigsäure ab. Dann nimmt man in Toluol auf und wascht die organische Phase mit Natronlauge und Wasser neutral, Man zieht das Toluol ab und destilliert den Rückstand im Vakuum. Man erhält 179 g (85,3 % der Theorie) 6-Oxa-1,1,2,3,3,8-hexamethyl-2,3,5,6,7,8-hexahydro-1H-benz[f]inden, das bei einem Druck von 4 mbar bei 155 bis 160°C übergeht.

**Beispiel 2**

a) Methylendiacetat

Zu einer Suspension von 300 g Paraformaldehyd in 1020 g Essigsäureanhydrid tropft man in 5 min 5 g konzentrierte Schwefelsäure, wobei die Temperatur von Raumtemperatur bis auf ca. 100°C ansteigt. Nach beendeter Zugabe wird auf 150°C erhitzt und 3 h bei dieser Temperatur gerührt. Dann kühlt man auf Raumtemperatur ab, gibt 25 g Natriumacetat zu und destilliert die flüchtigen Bestandteile im Vakuum ab. Man erhält dabei ca. 1300 g Destillat, das anschließend unter Zusatz von 10 g Natriumacetat über eine 50 cm Füllkörperkolonne fraktioniert wird. Dabei erhält man 1150 g (87,1 % der Theorie) Methylendiacetat vom Siedepunkt 96 bis 99°C bei 90 mbar.

b) 6-Oxa-1,1,2,3,3,8-hexamethyl-2,3,5,6,7,8-hexahydro-1H-benz[f]inden

Man gibt 200 g 2-(1',1',2',3',3'-Pentamethyl-5'-indanyl)-1-propanol, 50 g 85 %ige Phosphorsäure und 122 g Methylendiacetat in einen Autoklaven und erhitzt unter Rühren 1 h auf 150°C. Man arbeitet auf wie in Beispiel 1 und erhält 172 g (82,0 % der Theorie) 6-Oxa-1,1,2,3,3,8-hexamethyl-2,3,5,6,7,8-hexahydro-1H-benz[f]inden.

**Beispiel 3**

a) Methylendiacetat
Siehe Beispiel 2 a).
b) 6-Oxa-1,1,2,3,3,8-hexamethyl-2,3,5,6,7,8-hexahydro-1H-benz[f]inden
Man gibt 200 g 2-(1',1',2',3',3'-Pentamethyl-5'-indanyl)-1-propanol, 6,8 g p-Toluolsulfonsäure und 122 g Methylendiacetat in einen Autoklaven und erhitzt unter Rühren 8 h auf 130° C. Man arbeitet auf wie in Beispiel 1 und erhält 167 g (79,6 % der Theorie) 6-Oxa-1,1,2,3,3,8-hexamethyl-2,3,5,6,7,8-hexahydro-1H-benz[f]inden.

**Beispiel 4**

6-Oxa-1,1,2,3,3,8-hexamethyl-2,3,5,6,7,8-hexahydro-1H-benz[f]inden
Man gibt 200 g 2-(1',1',2',3',3'-Pentamethyl-5'-indanyl)-1-propanol in einen Autoklaven und erwärmt auf 70° C. Dann gibt man 50 g 85 %ige Phosphorsäure und anschließend 27,6 g Paraformaldehyd zu. Dann werden 120 g Propionsäureanhydrid zugetropft. Nach beendeter Zugabe wird der Autoklav verschlossen, und man rührt 5 h bei 140° C. Nach beendeter Reaktion destilliert man die entstandene Propionsäure ab und arbeitet wie in Beispiel 1 auf. Man erhält 171 g (81,5 % der Theorie) 6-Oxa-1,1,2,3,3,8-hexamethyl-2,3,5,6,7,8-hexahydro-1H-benz[f]inden.

**Beispiel 5**

6-Oxa-1,1,3,8-tetramethyl-3-ethyl-2,3,5,6,7,8-hexahydro-1H-benz[f]inden und 6-Oxa-1,3,3,8-tetramethyl-1-ethyl-2,3,5,6,7,8-hexahydro-1H-benz[f]inden
Man gibt 200 g eines Gemisches aus 2-(1',1',3'-Trimethyl-3'-ethyl-5'-indanyl)-1-propanol und 2-(1',3',3'-Trimethyl-1'-ethyl-5'-indanyl)-1-propanol (herzustellen durch Reaktion von Propylenoxid mit 1,1,3-Trimethyl-3-ethylindan), 20 g sauer dotierter Montmorillonit (K10-Katalysator (Fa. Süd-Chemie, München)), 27,6 g Paraformaldehyd und 94 g Essigsäureanhydrid in einen Autoklaven und erhitzt 5 h auf 150° C. Man läßt auf Raumtemperatur abkühlen, filtriert den Katalysator ab, zieht die entstandene Essigsäure ab und destilliert den Rückstand im Vakuum. Man erhält 175 g (83,4 % der Theorie) eines Gemisches aus 6-Oxa-1,1,3,8-tetramethyl-3-ethyl-2,3,5,6,7,8-hexahydro-1H-1H-benz[f]inden und 6-Oxa-1,3,3,8-tetramethyl-1-ethyl-2,3,5,6,7,8-hexahydro-1H-benz[f]inden.

**Beispiel 6**

2-Oxa-1,2,3,4-tetrahydro-naphthalin
Man gibt 94 g Essigsäureanhydrid, 50 g 85 %ige Phosphorsäure, 27,6 g Paraformaldehyd und 100 g 2-Phenylethanol in einen Autoklaven und erhitzt 5 h auf 130° C. Man arbeitet auf wie in Beispiel 1 und erhält 89 g (81,0 % der Theorie) 2-Oxa-1,2,3,4-tetrahydro-naphthalin vom Siedepunkt 55° C bei 1,7 mbar.

**Patentansprüche**

1) Verfahren zur Herstellung von Isochroman-Derivaten durch Umsetzung von Phenylethylalkoholen der Formel I

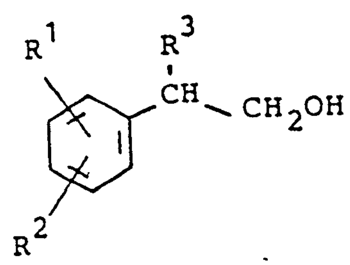

(I)

in der
$R^1$ und $R^2$ gleich oder verschieden sind und Wasserstoff, $C_1$-$C_6$-Alkyl oder $C_1$-$C_6$-Alkoxy bedeuten oder gemeinsam einen gegebenenfalls durch $C_1$-$C_6$-Alkyl substituierten Cyclopentan- oder Cyclohexanring bilden und

$R^3$ Wasserstoff oder $C_1$-$C_6$-Alkyl bedeutet,
mit methylenierend-cyclisierend wirkenden Agentien in Gegenwart saurer Katalysatoren bei erhöhter Temperatur, dadurch gekennzeichnet, das man als methylenierend-cyclisierend wirkendes Agens Formaldehyd und ein Carbonsäureanhydrid der Formel II

$$R^4-\overset{O}{\overset{\|}{C}}-O-\overset{O}{\overset{\|}{C}}-R^4 \qquad (II)$$

in der
$R^4$ für einen $C_1$-$C_6$-Alkyltest steht, oder
einen Methylen-diester der Formel (III)

$$R^4-\overset{O}{\overset{\|}{C}}-O-CH_2-O-\overset{O}{\overset{\|}{C}}-R^4 \qquad (III)$$

in der
$R^4$ für einen $C_1$-$C_6$-Alkylrest steht, verwendet.

2) Verfahren nach Anspruch 1, dadurch gekennzeichnet, daß man als Carbonsäureanhydrid Essigsäureanhydrid verwendet.

3) Verfahren nach Anspruch 1, dadurch gekennzeichnet, daß man als Methylen-diester Methylen-diacetat verwendet.

4) Verfahren nach den Ansprüchen 1 bis 3, dadurch gekennzeichnet, daß man als sauren Katalysator Orthophosphorsäure oder einen sauren Ton verwendet.

5) Verfahren nach den Ansprüchen 1 bis 4, dadurch gekennzeichnet, daß das Verfahren im Temperaturbereich von Raumtemperatur bis etwa 200°C durchgeführt wird.

**Claims**

1. Process for the preparation of isochroman deriva by reacting phenylethyl alcohols of the formula 1

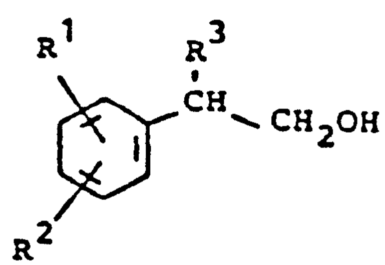

(I)

in which
$R^1$ and $R^2$ are identical or different and denote hydrogen, $C_1$-$C_6$-alkyl or $C_1$-$C_6$-alkoxy or together form an optionally $C_1$-$C_6$-alkyl-substituted cyclopentane or cyclohexane ring and
$R^3$ denotes hydrogen or $C_1$-$C_6$-alkyl,
with methylenating-cyclising agents in the presence of acid catalysts at an elevated temperature, characterised in that formaldehyde and a carboxylic acid anhydride of the formula II

$$R^4-\overset{O}{\overset{\|}{C}}-O-\overset{O}{\overset{\|}{C}}-R^4 \qquad (II)$$

in which
$R^4$ represents a $C_1$-$C_6$-alkyl radical, or a methylene diester of the formula (III)

$$R^4-\overset{O}{\overset{\|}{C}}-O-CH_2-O-\overset{O}{\overset{\|}{C}}-R^4 \qquad (III)$$

in which

$R^4$ represents a $C_1$-$C_6$-alkyl radical, are used as the methylenating-cyclising agent.

2. Process according to Claim 1, characterised in that acetic anhydride is used as the carboxylic acid anhydride.

3. Process according to Claim 1, characterised in that methylene diacetate is used as the methylene diester.

4. Process according to Claims 1 to 3, characterised in that orthophosphoric acid or an acid clay are used as the acid catalyst.

5. Process according to Claims 1 to 4, characterised in that the process is carried out in the temperature range from room temperature to about 200° C.

**Revendications**

1 - Procédé de préparation de dérivés de l'isochromane par réaction d'alcools phényléthyliques de formule I:

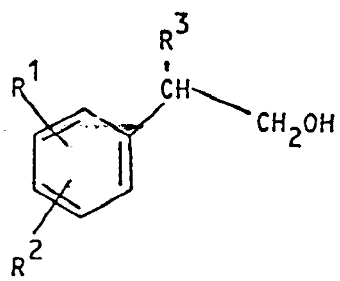

(I)

dans laquelle

$R^1$ et $R^2$, ayant des significations identiques ou différentes, représentent chacun l'hydrogène, un groupe alkyle en $C_1$-$C_6$ ou alcoxy en $C_1$-$C_6$ ou forment ensemble un noyau cyclopentane ou cyclohexane éventuellement substitué par des groupes alkyle en $C_1$-$C_6$, et

$R^3$ représente l'hydrogène ou un groupe alkyle en $C_1$-$C_6$, avec des agents méthylénants-cyclisants en présence de catalyseurs acides, à température élevée, caractérisé en ce que l'on utilise en tant qu'agents méthylénants-cyclisants le formaldéhyde et un anhydride d'acide carboxylique de formule II:

$$R^4-\overset{O}{\overset{\|}{C}}-O-\overset{O}{\overset{\|}{C}}-R^4 \qquad (II)$$

dans laquelle

$R^4$ représente un groupe alkyle en $C_1$-$C_6$, ou bien un diester de méthyléne de formule III

$$R^4-\overset{O}{\overset{\|}{C}}-O-CH_2-\overset{O}{\overset{\|}{O}}-C-R^4 \qquad (III)$$

dans laquelle

$R^4$ représente un groupe alkyle en $C_1$-$C_6$.

2 - Procédé selon la revendication 1, caractérisé en ce que l'anhydride d'acide carboxylique utilisé est l'anhydride acétique.

3 - Procédé selon la revendication 1, caractérisé en ce que le diester de méthylène utilisé est le diacétate de méthylène.

4 - Procédé selon les revendications 1 à 3, caractérisé en ce que l'on utilise en tant que catalyseur acide l'acide orthophosphorique ou une argile acide.

5 - Procédé selon les revendications 1 à 4, caractérisé en ce que l'on opère dans un intervalle de température allant de la température ambiante jusqu'à 200° C environ.